(19) Europäisches Patentamt / European Patent Office / Office européen des brevets

(11) **EP 2 075 018 B1**

(12) **FASCICULE DE BREVET EUROPEEN**

(45) Date de publication et mention
de la délivrance du brevet:
**13.02.2013  Bulletin 2013/07**

(51) Int Cl.:
*A61M 1/02* *(2006.01)*      *A61M 1/36* *(2006.01)*
*B01D 39/16* *(2006.01)*

(21) Numéro de dépôt: **08291171.0**

(22) Date de dépôt: **11.12.2008**

(54) **Procédé de greffage d'un élément poreux pour la déleucocytation**

Verfahren zum Okulieren eines porösen Elements für die Leukozytenentfernung

Method for grafting a porous element for leukodepletion

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité:  **21.12.2007  FR 0709088**

(43) Date de publication de la demande:
**01.07.2009  Bulletin 2009/27**

(73) Titulaires:
• **MACO PHARMA**
  **59420 Mouvaux (FR)**
• **Centre National de la Recherche Scientifique (CNRS)**
  **75794 Paris Cedex 16 (FR)**

(72) Inventeurs:
• **Marmey, Pascal**
  **59126 Linselles (FR)**
• **Bessy, Emilie**
  **59800 Lille (FR)**
• **Lutz, Pierre**
  **67800 Bischheim (FR)**
• **Henard, Grégory**
  **59000 Lille (FR)**

(74) Mandataire: **Sayettat, Julien Christian**
  **STRATO-IP**
  **18, rue Soleillet**
  **75020 Paris (FR)**

(56) Documents cités:
**EP-A- 0 561 379      EP-A- 0 606 646**
**EP-A- 0 811 412      FR-A- 2 892 949**

**Description**

L'invention concerne un procédé de greffage d'un élément poreux pour la déleucocytation, ainsi que l'élément poreux obtenu par ce procédé, une unité de filtration comprenant un tel élément poreux et un système à poches comprenant une telle unité de filtration.

L'invention s'applique typiquement à la filtration du sang ou d'un composant sanguin tel qu'un sang total ou un concentré de globules rouges ainsi qu'à la séparation et au recueil des composants sanguins, notamment en circuit clos.

Les leucocytes ont des effets indésirables très importants, ce qui a conduit à chercher à les éliminer des composants sanguins destinés à la transfusion. En effet, les leucocytes augmentent les risques de rejet immunitaire tel que la maladie du greffon contre l'hôte et favorisent la transmission d'agents infectieux.

Pour éliminer les leucocytes des composants sanguins destinés à la transfusion, on connaît déjà des unités de filtration comprenant une enveloppe renfermant un milieu de déleucocytation. Dans de telles unités, le milieu de déleucocytation comprend une ou plusieurs membrane(s) et/ou une ou plusieurs couche(s) de non tissé réalisée(s) en un matériau polymère et traitée(s) de façon à améliorer le taux de déleucocytation, la récupération des composants sanguins, le temps d'amorçage de la filtration et/ou la sélectivité de la filtration, par exemple en laissant passer les plaquettes.

Par exemple, le document EP-A1-0 606 646 décrit une unité de filtration dont le milieu de déleucocytation est revêtu d'un polymère comprenant des groupements fonctionnels basiques et des groupements hydrophiles non ioniques, tels que des groupements poly(éthylène glycol), la proportion entre les groupements basiques et les groupements hydrophiles étant choisie pour obtenir un faible taux de leucocytes résiduels et un bon rendement en récupération des globules rouges.

Dans ce document, le revêtement est réalisé en deux étapes. Ces étapes consistent en la copolymérisation radicalaire d'un monomères basique et d'un monomère hydrophile, puis la simple imprégnation du milieu de déleucocytation avec une solution du copolymère obtenu. Il en résulte que le revêtement polymère n'est pas lié de façon covalente au milieu de déleucocytation, provoquant un risque d'élution du copolymère dans le filtrat. En outre, la polymérisation radicalaire nécessite l'emploi d'un amorceur de polymérisation qui peut aussi se retrouver dans le filtrat, ce qui n'est pas recommandé.

De même, le document US-20060207937 décrit une unité de filtration destinée à éliminer les leucocytes d'un sang total comprenant un milieu de déleucocytation revêtu d'un polymère obtenu par réaction d'un monomère hydrophobe et d'un monomère hydrophile. Là encore, le revêtement polymère n'est pas lié de façon covalente à la surface du milieu de déleucocytation.

Enfin, dans le document WO2007/054638, le milieu de déleucocytation de l'unité de filtration destinée à éliminer les leucocytes en laissant passer les plaquettes est imprégné d'un copolymère à blocs composé d'un bloc hydrophile de type poly(oxyde d'éthylène) et de deux blocs hydrophobes.

Dans tous ces documents, il apparaît que le revêtement d'un filtre à déleucocyter est composé d'un copolymère à bloc comportant des parties hydrophiles et non hydrophiles.

Dans le document EP-A-811412, l'élément poreux est imprégné avec une solution comprenant un haut polymère ayant des groupes fonctionnels hydrophiles non ioniques.

Contrairement à l'enseignement de documents antérieurs, la demanderesse a mis au point un procédé de greffage d'un élément poreux pour la déleucocytation à l'aide d'un unique polymère hydrophile, sans utiliser de polymère ou bloc hydrophobe ou basique. En outre, le procédé permet d'assurer le greffage irréversible du polymère sur l'élément poreux.

De façon surprenante, une unité de filtration comprenant un tel élément poreux possède des performances améliorées, telles qu'un plus petit temps d'amorçage, un temps de filtration plus court et un meilleur taux de récupération des composants sanguins d'intérêts, tout en conservant un bon taux de rétention des leucocytes.

Selon un premier aspect, l'invention propose un procédé de greffage d'un élément poreux pour la déleucocytation par adsorption et/ou filtration d'un fluide biologique tel que le sang ou un composant sanguin, ledit procédé comprenant les étapes prévoyant :

- d'imprégner l'élément poreux avec une solution contenant un poly(oxyde d'éthylène) ayant des groupements terminaux fonctionnels identiques ou différents;
- d'appliquer un traitement ionisant sur l'élément poreux imprégné de sorte à assurer un pontage covalent entre le poly(oxyde d'éthylène) et ledit élément poreux.

Selon un second aspect, l'invention propose un élément poreux pour la déleucocytation par adsorption et/ou filtration d'un fluide biologique tel que le sang ou un composant sanguin, ledit élément ayant été greffé avec un poly (oxyde d'éthylène) par mise en oeuvre du procédé selon le premier aspect de l'invention, une extrémité dudit poly(oxyde d'éthylène) étant liée de façon covalente audit élément poreux.

Un troisième aspect de l'invention concerne une unité de filtration d'un fluide biologique tel que le sang ou un composant sanguin comprenant une enveloppe extérieure munie d'au moins un orifice d'entrée et d'au moins un orifice de sortie, l'enveloppe renfermant un milieu de déleucocytation interposé entre lesdits orifices, ledit milieu de déleucocytation comprenant au moins un élément poreux selon le deuxième aspect de l'invention.

Enfin, les quatrième et cinquième aspects de l'invention concernent un système à poches pour la déleucocytation d'un fluide biologique tel que le sang ou le composant sanguin comprenant une unité de filtration selon le troisième aspect de l'invention connectée ou connectable à une poche de collecte du fluide biologique.

D'autres objets et avantages apparaîtront au cours de la description qui suit.

La figure 1 représente la structure chimique d'un monométhacrylate de méthoxypoly(oxyde d'éthylène), dans laquelle n est un entier supérieur ou égal à 2.

La figure 2 représente un schéma de principe de la synthèse d'un monométhacrylate de méthoxypoly(oxyde d'éthylène) à partir d'un méthoxypoly(oxyde d'éthylène).

La figure 3 représente de façon schématique les étapes du greffage d'un élément poreux avec un monométhacrylate de méthoxypoly(oxyde d'éthylène).

La figure 4 représente de façon schématique un équipement permettant de réaliser l'imprégnation d'un élément poreux selon le principe de foulardage séchage.

La figure 5 représente de façon schématique la structure d'un monométhacrylate de méthoxypoly(oxyde d'éthylène) greffé à la surface d'un élément poreux.

Les figures 6 et 7 représentent chacune de façon schématique un système à poches comprenant une unité de filtration selon l'invention.

L'invention propose un procédé de greffage d'un élément poreux pour la déleucocytation par adsorption et/ou filtration d'un fluide biologique tel que le sang ou un composant sanguin.

L'élément poreux pour la déleucocytation par adsorption et/ou filtration est par exemple formé d'une couche poreuse ou fibreuse réalisée en polyoléfine tel que le polypropylène ou en polyester tel que le polyéthylène téréphtalate ou polybutylène téréphtalate.

La couche poreuse fibreuse est notamment une couche de nontissé de type meltblown.

En particulier, le diamètre moyen des pores de l'élément poreux est compris entre 3 et 20 $\mu$m.

Cet élément poreux est apte à éliminer les leucocytes d'un fluide biologique tel que le sang ou un composant sanguin. Le composant sanguin est notamment le sang total ou un concentré de globules rouges obtenu à partir d'un don de sang total.

Afin d'améliorer l'hydrophilie et l'hémocompatibilité de l'élément poreux de déleucocytation, sa surface peut être recouverte par des molécules ou macromolécules spécifiques qui sont ensuite liées de façon permanente afin d'éviter le relargage dans les produits sanguins.

Selon l'invention, l'élément poreux est greffé selon un procédé comprenant les étapes prévoyant :

- d'imprégner l'élément poreux avec une solution contenant un poly(oxyde d'éthylène) ayant des groupements terminaux fonctionnels identiques ou différents ;
- d'appliquer un traitement ionisant sur l'élément poreux imprégné de sorte à assurer un pontage covalent entre le poly(oxyde d'éthylène) et ledit élément poreux.

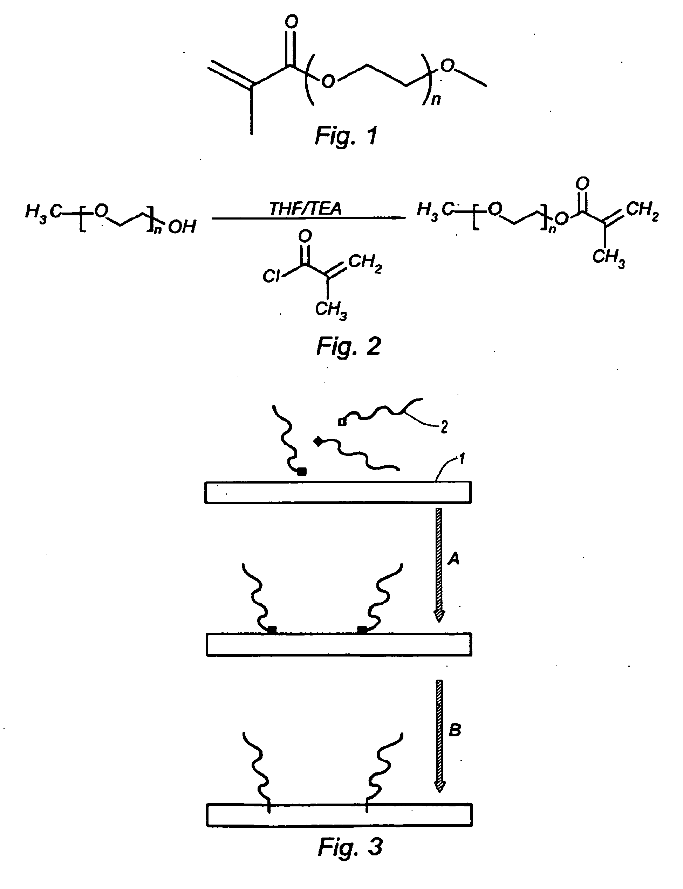

La figure 3 représente de façon schématique les étapes de ce procédé par lequel un élément poreux 1 est greffé avec un poly(oxyde d'éthylène) 2 par une étape d'imprégnation A et de greffage B.

Les poly(oxyde d'éthylène) utiles pour l'invention sont hydrophiles et possèdent une structure linéaire ou ramifiée, et avantageusement linéaire. Par exemple, le poly(oxyde d'éthylène) possède la formule $R_1$-(CH$_2$-CH$_2$-O)$_{n-1}$-CH$_2$-CH$_2$-R$_2$ (I), dans laquelle n est un entier supérieur ou égal à 2 et $R_1$ et $R_2$ représentent des groupements fonctionnels identiques ou différents. Notamment, $R_1$ et $R_2$ représentent un groupement fonctionnel choisi parmi un hydroxy, un alkoxy, par exemple un méthoxy, un phénoxy un benzoxy ou groupement terminal qui est polymérisable sous traitement ionisant.

Des polymères particuliers sont les méthoxypoly(oxyde d'éthylène) de formule (I), dans laquelle $R_1$ est méthoxy et $R_2$ est hydroxy ou les diméthoxypoly(oxyde d'éthylène) de formule (I), dans laquelle $R_1$ et $R_2$ sont des méthoxy.

D'autres polymères particuliers sont les macromonomères de poly(oxyde d'éthylène), notamment ceux de formule (I), dans laquelle l'un au moins de $R_1$ ou $R_2$ est un groupement terminal qui est polymérisable sous traitement ionisant.

Les macromonomères sont des polymères ou oligomères ayant une extrémité réactive, par exemple un groupement fonctionnel polymérisable, qui lui permet de réagir comme un monomère.

Le macromonomère de poly(oxyde d'éthylène) selon l'invention comprend un groupement terminal qui est polymérisable sous traitement ionisant. Ce groupement polymérisable favorise le pontage covalent avec l'élément poreux.

Des exemples de groupements terminaux polymérisables par traitement ionisant sont un acryloyloxy, un méthacryloyloxy, un acrylamido, un méthacrylamido, un vinyloxy ou un allyloxy.

L'autre extrémité du macromonomère de poly(oxyde d'éthylène) porte soit un groupement non polymérisable tel qu'un alkoxy, par exemple un méthoxy, un phénoxy ou un benzoxy, soit un groupement polymérisable par traitement ionisant qui peut être le même ou non que celui porté à la première extrémité.

Une famille particulière de macromonomères de poly(oxyde d'éthylène) est représentée sur la figure 1 par sa formule semi-développée dans laquelle n est un entier supérieur ou égal à 2. Ces macromonomères comprennent un oligomère ou polymère de poly(oxyde d'éthylène) ayant à l'une de ses extrémités un méthacrylate en tant que groupement polymérisable, et à l'autre extrémité un groupement méthoxy.

Ces macromonomères de poly(oxyde d'éthylène), possèdent des propriétés intéressantes. En effet, la partie polymère poly(oxyde d'éthylène) possède à la fois des propriétés d'hémocompatibilité et d'hydrophilie, et la fonction méthacrylate peut être activée par apport d'énergie afin de permettre le pontage covalent du macromonomère sur l'élément poreux de déleucocytation.

Des exemples particuliers de macromonomères de poly(oxyde d'éthylène) sont les monométhacrylates d'hydroxypoly(oxyde d'éthylène) de formule (I), dans laquelle $R_1$ est hydroxy et $R_2$ est méthacryloyloxy, les diméthacrylates de poly(oxyde d'éthylène), de formule (I), dans laquelle $R_1$ et $R_2$ sont des méthacryloyloxy, et les monométhacrylates de méthoxypoly(oxyde d'éthylène) de formule (I), dans laquelle $R_1$ est méthoxy et $R_2$ est méthacryloyloxy.

Les monométhacrylates de méthoxypoly(oxyde d'éthylène) sont disponibles dans le commerce (Sigma-Aldrich).

Les monométhacrylates de méthoxypoly(oxyde d'éthylène) et les diméthacrylates de poly(oxyde d'éthylène) sont synthétisables à partir des hydroxy, méthoxypoly(oxyde d'éthylène) et dihydroxypoly(oxyde d'éthylène), respectivement, par réaction avec un chlorure de méthacryloyle dans un solvant tétrahydrofurane en présence de triéthylamine (Figure 2 en relation avec les monométhacrylates de méthoxypoly(oxyde d'éthylène)).

Une autre voie de synthèse est décrite dans le document Biomacromolecules 2004, 5, 1280-1287. Brièvement et en relation avec le diméthacrylate de poly(oxyde d'éthylène) de masse molaire 2960 g/mol, la synthèse est la suivante : à une solution de 10 g (3,4mmol.) de poly(oxyde d'éthylène) purifié 2960g/mol dans 50mL de dichlorométhane sont ajoutés 0,7mL (4,8mmol, 0,7eq. par OH) de triéthylamine et 1,2mL (7,4mmol, 1,1eq. par OH) d'anhydride méthacrylique 94%. Le mélange réactionnel est laissé sous agitation et à température ambiante pendant 4 jours et demi. Une purification est effectuée par filtration sur alumine neutre (gradient d'élution dichlorométhane/méthanol : 70/30 à 50/50). Les solvants sont évaporés et le résidu est dissous à 30°C dans 80mL de diméthylsulfoxyde. Le macromonomère est alors précipité de sa solution à température ambiante et sous agitation dans 1,2 L d'éther puis lavé à l'éther avant d'être filtré sur verre fritté (porosité 4). Le produit final est récupéré après séchage sous vide jusqu'à poids constant dans une étuve, 9,5g de solide blanc.

Les méthoxypoly(oxyde d'éthylène) et les monométhacrylates de méthoxypoly(oxyde d'éthylène) sont hydrosolubles, ce qui facilite leur manipulation.

En particulier, la masse molaire du poly(oxyde d'éthylène) est supérieure ou égale à 500 g/mol, notamment comprise entre 800 et 2000 g/mol.

En dessous de 500 g/mol, les poly(oxyde d'éthylène) sont généralement toxiques et déconseillés pour un contact avec le sang ou un composant sanguin.

Au dessus de 5 000 g/mol, la chaîne pendante des poly(oxyde d'éthylène) greffés à la surface d'un élément poreux forme une pelote statistique qui possède une capacité de répulsion stérique. Cette dernière réduit l'adsorption des protéines et des cellules à la surface de l'élément poreux, y compris celle des leucocytes.

La solution d'imprégnation est un mélange constitué d'un poly(oxyde d'éthylène) en tant que soluté et d'un liquide aqueux et/ou organique en tant que solvant. Elle contient une faible concentration de poly(oxyde d'éthylène). Notamment, les poly(oxyde d'éthylène) sont dissous dans un solvant à des concentrations comprises entre 0,01 et 5 % en masse, en particulier comprises entre 0,1 et 2% en masse.

Les solvants d'imprégnation sont notamment l'eau, l'éthanol et l'acétone, purs ou mélangés dans différentes proportions. L'eau possède l'avantage sur les solvants organiques d'être non toxique.

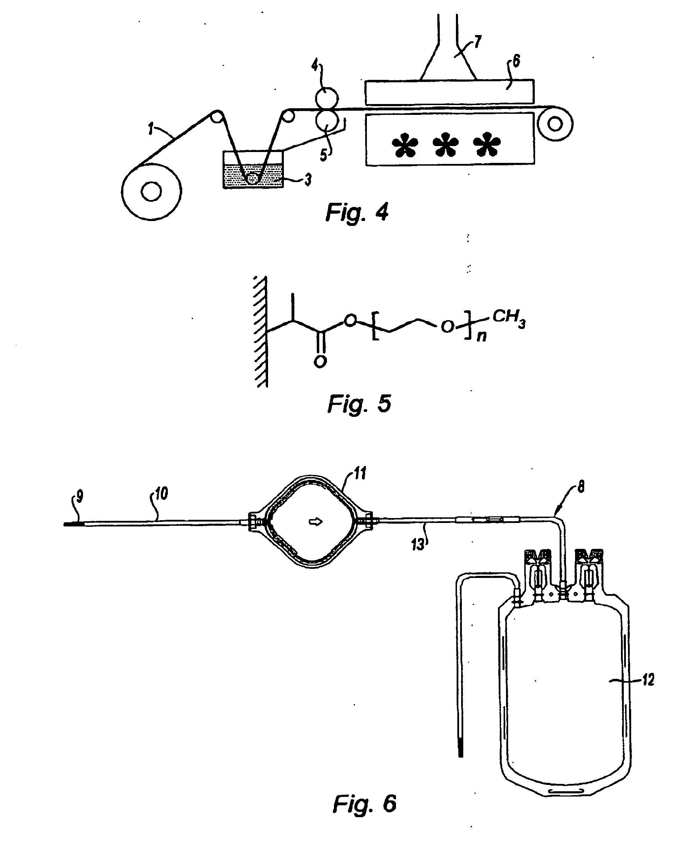

L'imprégnation est réalisée selon un principe de foulardage séchage, par exemple avec un équipement représenté sur la figure 4. L'élément poreux 1 est trempé dans une solution 3 de poly(oxyde d'éthylène). Le surplus de solution imprégnée dans l'élément poreux 1 est ensuite exprimé ou essoré en passant entre deux rouleaux 4,5 dont la pression est comprise entre 1 et 5 bars. Puis l'élément poreux 1 est convoyé dans un four 6 équipé d'une ventilation mécanique 7 afin de le sécher par évaporation du solvant. La vitesse, comprise entre 1 et 10 m/min, est régulée en fonction de la nature et de la quantité de solvant emporté par l'élément poreux.

On détermine un taux d'emport par pesée de l'élément poreux avant imprégnation et après imprégnation et séchage selon la formule suivante.

**Taux d'emport (%) = (Masse élément poreux imprégné et séché - Masse élément poreux avant imprégnation) / Masse élément poreux avant imprégnation.**

En particulier, le taux d'emport est compris entre 0,1 et 5% en poids de l'élément poreux.

Le poly(oxyde d'éthylène) est ensuite lié de façon covalente à l'élément poreux par application d'un traitement ionisant.

Dans le cas où le poly(oxyde d'éthylène) est un macromonomère de poly(oxyde d'éthylène), le macromonomère est greffé à l'élément poreux pour obtenir le pontage covalent. La polymérisation est réalisée notamment par activation du groupement terminal polymérisable du macromonomère. Elle est obtenue par exemple par application d'un traitement ionisant sur l'élément poreux imprégné.

Le traitement ionisant apporte l'énergie suffisante pour provoquer la rupture d'une liaison chimique et en créer une nouvelle. Il peut être de différente nature tel qu'un traitement au plasma avec un gaz tel que l'argon, azote, l'oxygène, ou bien une irradiation par rayonnements ionisants ou faisceaux d'électrons tel que les rayons gamma ou bêta.

L'activation a pour but de greffer de façon covalente le poly(oxyde d'éthylène) en surface afin de l'immobiliser et d'éviter le relargage du polymère de poly(oxyde d'éthylène) dans le fluide filtré au passage du produit sanguin dans l'unité de filtration.

Avantageusement, le procédé de greffage ne comprend pas d'étape préalable d'irradiation de l'élément poreux. En effet, cette étape n'est pas nécessaire pour obtenir le greffage et l'imprégnation d'un poly(oxyde d'éthylène).

La figure 5 représente la structure chimique d'un macromonomère de poly(oxyde d'éthylène) une fois greffé sur l'élément poreux.

Afin de contrôler l'efficacité du procédé de greffage, des extractions au Soxhlet sont réalisées sur des échantillons à différentes étapes de la réalisation du greffage. L'extraction sur l'élément poreux après traitement ionisant permet la détermination de l'extractible contenu dans l'élément poreux non traité. L'extraction sur l'élément poreux imprégné avant et après traitement ionisant permet de déterminer l'efficacité d'un tel traitement.

Le taux de greffage est ensuite déterminé par la formule suivante :

**Taux de greffage = (masse d'extractible sans traitement – masse d'extractible après traitement)/ Masse de l'élément poreux sans traitement**

Le rendement se calcule ensuite par la formule :

**Rendement de greffage = taux de greffage/ taux d'emport**

Le procédé selon l'invention permet d'obtenir un rendement de greffage supérieur à 70%.

Selon un deuxième aspect, l'invention propose un élément poreux pour la déleucocytation par adsorption et/ou filtration d'un fluide biologique tel que le sang ou un composant sanguin, ledit élément ayant été greffé avec un poly(oxyde d'éthylène) par mise en oeuvre du procédé selon le premier aspect de l'invention, une extrémité dudit poly(oxyde d'éthylène) étant liée de façon covalente audit élément poreux.

Cet élément poreux greffé avec le poly(oxyde d'éthylène) possède une tension superficielle critique de mouillabilité CWST (critical wetting surface tension) comprise entre 80 et 110 mN/m.

Cette CWST est déterminée par la méthode décrite dans le document WO-8903717.

Cette CWST très élevée, supérieure à 72 mN/m, démontre la bonne hydrophilie de l'élément poreux de déleucocytation, c'est-à-dire qu'il peut être mouillé par l'eau. La mouillabilité de l'élément poreux ainsi améliorée génère des temps d'amorçage de la filtration plus courts qu'avec des éléments poreux non greffés.

De façon surprenante, malgré cette mouillabilité élevée, le taux de déleucocytation d'une unité de filtration comprenant un tel élément poreux greffé est sensiblement le même qu'avec une unité de filtration comprenant un élément poreux non greffé.

L'invention concerne selon un troisième aspect une unité de filtration d'un fluide biologique tel que le sang ou un composant sanguin. Le composant sanguin est notamment le sang total ou un concentré de globules rouges obtenu à partir d'un don de sang total.

L'unité de filtration comprend une enveloppe extérieure munie d'au moins un orifice d'entrée et d'au moins un orifice de sortie, l'enveloppe renfermant un milieu de déleucocytation interposé entre lesdits orifices de sorte à former un compartiment d'entrée pour le fluide à filtrer et un compartiment de sortie du fluide filtré. Le milieu de déleucocytation comprend au moins un élément poreux greffé avec un poly(oxyde d'éthylène).

Selon une réalisation particulière, l'unité de filtration comprend en outre un pré-filtre et/ou post-filtre qui sont réalisés sous la forme d'au moins une couche de non-tissé et qui sont disposés respectivement côté amont et côté aval du milieu de déleucocytation.

Ces pré-filtres ou post-filtres ont notamment une taille de pores moyenne supérieure à celle du milieu de déleucocytation, par exemple comprise entre 25 et 50 $\mu$m.

Cette unité de filtration est utilisée pour la déleucocytation d'un produit sanguin, notamment les concentrés globulaires appauvris, c'est-à-dire préparés par centrifugation dure d'une unité de sang total.

Pour cela, l'unité de filtration est intégrée dans un système à poches.

En relation avec la figure 6 et selon un quatrième aspect, l'invention propose un système à poches 8 pour la déleucocytation d'un fluide biologique comprenant :

- un connecteur 9 destiné à être relié à une poche de collecte contenant le fluide biologique à filtrer, ledit connecteur étant connecté, par l'intermédiaire d'une tubulure 10, à un orifice d'entrée d'une unité de filtration 11 selon l'invention; et
- une poche de recueil 12 du filtrat, ladite poche étant reliée, par l'intermédiaire d'une tubulure 13 et au niveau d'un orifice d'entrée, à un orifice de sortie de l'unité de filtration.

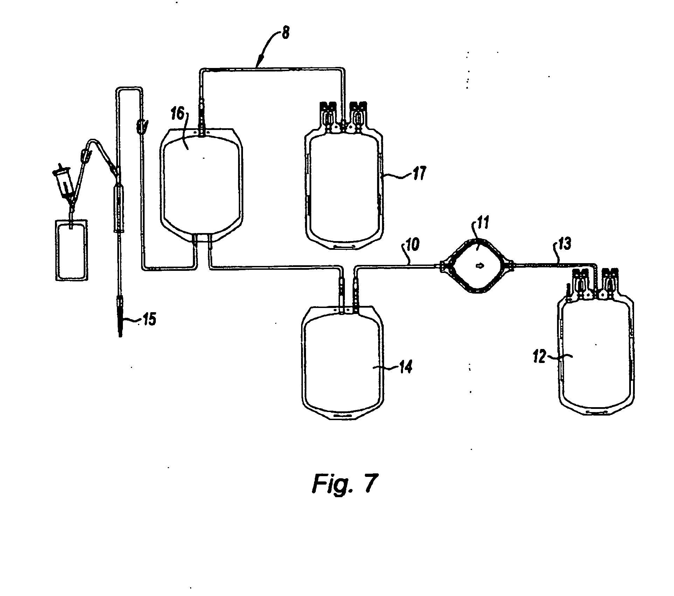

En variante représentée sur la figure 7 et selon un autre aspect de l'invention, le système à poches 8 comprend :

- une poche de collecte 14 du fluide biologique, ladite poche étant reliée, par l'intermédiaire d'une tubulure 10 et au niveau d'un orifice de sortie, à un orifice d'entrée d'une unité de filtration 11 selon l'invention, et
- une poche de recueil 12 du filtrat, ladite poche étant reliée, par l'intermédiaire d'une tubulure 13 et au niveau d'un orifice d'entrée, à un orifice de sortie de ladite unité de filtration 11.

Dans cette variante, le système à poches peut comprendre également des moyens de prélèvement du sang, tel qu'une aiguille, ainsi que des poches secondaires 16,17 reliées par des tubulures pour la séparation et le recueil du sang total et de ses composants.

Pour réaliser la filtration d'un concentré de globules rouges obtenu par centrifugation d'une unité de sang total, on utilise l'un ou l'autre des systèmes à poches. Avantageusement, une solution de conservation (SAGM, PAGSM) est placée dans la poche 12 de recueil du filtrat.

Selon une réalisation particulière, avant d'effectuer la filtration, la solution de conservation est transférée extemporanément, à travers l'unité de filtration 11, vers la poche de collecte 14 contenant le concentré globulaire afin de pré-mouiller le milieu de déleucocytation et de diluer le concentré de globules rouges. La filtration du concentré de globules rouges avec la solution de conservation peut alors commencer.

Le milieu de déleucocytation de l'unité de filtration reste donc à l'état sec jusqu'à son utilisation.

Exemples

Préparation des solutions d'imprégnation.

Les monométhacrylates de méthoxypoly(oxyde d'éthylène) de masse molaire 300, 475 et 1100 g/mol (Sigma-Aldrich) et le diméthoxypoly(oxyde d'éthylène) de masse molaire 1000 g/mol (Sigma-Aldrich) sont utilisés en l'état. Les monométhacrylates de méthoxypoly(oxyde d'éthylène) de masse molaire supérieure à 1100 g/mol ont été synthétisés à l'institut Charles Sadron du CNRS par fonctionnalisation du méthoxypoly(oxyde d'éthylène) (Figure 2).

Les monométhacrylates de méthoxypoly(oxyde d'éthylène) ou le diméthoxypoly(oxyde d'éthylène) sont dissous dans trois types de solvants : de l'eau, un mélange éthanol/eau à différents rapports (V/V), un mélange éthanol/acétone à différents ratios (V/V).

Les solutions suivantes ont été préparées :

|  | Solvant | Concentration |
|---|---|---|
| Solution 1 | Eau | 0,5% |
| Solution 2 | Ethanol / eau (20/80) | 0,2% |
| Solution 3 | Ethanol / eau (50/50) | 0,2% |
| Solution 4 | Ethanol / eau (20/80) | 0,5 % |
| Solution 5 | Ethanol / eau (50/50) | 0,8 % |
| Solution 6 | Ethanol/ acétone(50/50) | 1 % |

Imprégnation

Deux types de couches de nontissé de poly(butylène téréphtalate) ont été modifiés par greffage d'un monométhacrylate de méthoxypoly(oxyde d'éthylène) de masse molaire 1100g/mol.

|  | Type 1 | Type 2 |
|---|---|---|
| Taille moyenne des fibres ($\mu$m) | 1-2 | 1-2 |
| Perméabilité à l'air (l/m$^2$/s - 196 Pa) | 120-140 | 260-300 |
| Taille moyenne des pores ($\mu$m) | 8-10 | 15-20 |
| Epaisseur moyenne ($\mu$m) | 220-280 | 220-280 |

L'imprégnation des deux types de couche a été réalisée selon le principe du foulardage séchage comme décrit ci-dessus avec les solutions préparées ci dessus.
Le taux d'emport obtenu est compris entre 0,4 % et 1,2 %.

Greffage

Le greffage des couches imprégnées de type 1 et 2 obtenues ci-dessus a été réalisé par traitement plasma dans les conditions suivantes :
Le traitement plasma est réalisé sur un plasma de décharge à haute fréquence. Le plasma est généré dans une enceinte à pression réduite (10 < P < 250 mTorr). La puissance du générateur est comprise entre 300 et 1400 W. Le gaz utilisé est un gaz inerte avec un débit compris entre 0,1 et 2 l/min. Le temps de traitement (temps de séjour dans le plasma) est compris entre 20 secondes et 2 minutes.
Après greffage, la CWST de la couche est comprise entre 90 et 105 mN/m.
Avec une couche de type 1 imprégnée avec une solution à 0,5% molaire de monométhacrylate de méthoxypoly (oxyde d'éthylène) de masse molaire 1100 g/mol dans un solvant eau/éthanol 50/50 puis traités au plasma, le taux d'emport obtenu est compris entre 0,7 et 1% et le rendement de greffage est supérieur à 70%.
Avec une couche de type 1 imprégnée avec une solution à 0,5% molaire de diméthoxypoly(oxyde d'éthylène) de masse molaire 1000 g/mol dans un solvant eau/éthanol 80/20 puis traités au plasma, le taux d'emport obtenu est de 0,76%, la CWST est de 90mN/m et le rendement de greffage est supérieur à 80%.

Filtration

Une unité de filtration (A) a été réalisée avec la composition suivante :

- deux couches de polyester
- deux couches de type 2 greffées avec un monométhacrylate de méthoxypoly(oxyde d'éthylène) de masse molaire 1100 g/mol,

- 26 couches de type 1 greffées avec un monométhacrylate de méthoxypoly(oxyde d'éthylène) de masse molaire 1100 g/mol, et
- une couche de polyester.

Une unité de filtration (B) a été réalisée avec la composition suivante :

- deux couches de polyester
- une couche de type 2 greffée avec un monométhacrylate de méthoxypoly(oxyde d'éthylène) de masse molaire 1100 g/mol,
- 25 couches de type 1 greffées avec un monométhacrylate de méthoxypoly(oxyde d'éthylène) de masse molaire 1100 g/mol, et
- une couche de polyester.

Une unité de filtration (C) a été réalisée avec la composition suivante :

- deux couches de polyester
- une couche de type 2 greffée avec un monométhacrylate de méthoxypoly(oxyde d'éthylène) de masse molaire 1100 g/mol,
- 25 couches de type 1 greffées avec un diméthoxypoly(oxyde d'éthylène) de masse molaire 1000 g/mol, et
- une couche de polyester.

Ces unités ont été utilisées pour la filtration d'un concentré de globules rouges obtenu par centrifugation d'une unité de sang total. La séparation et la filtration sont réalisées dans un système à poches tel que représenté sur la figure 6.

La poche de recueil du filtrat contient une solution de conservation (SAGM, PAGSM). Avant d'effectuer la filtration, la solution de conservation est transférée extemporanément, à travers l'unité de filtration, vers le concentré globulaire afin de pré-mouiller le milieu de déleucocytation et diluer le concentré de globules rouges. La filtration du concentré de globules rouges avec la solution de conservation peut alors commencer.

Les résultats obtenus sont moyennés sur 5 essais.

Résultats

Tableau 1

| | Temps d'amorçage (minutes: secondes) | Temps de filtration (minutes) | Nombre de leucocytes résiduels |
|---|---|---|---|
| Unité A | 1:00 | 21 | $5{,}03.10^4$ |
| Unité B | 0:49 | 14,8 | $2{,}58.10^5$ |
| Unité C | 1:04 | 24 | $9{,}3.10^4$ |

La CWST élevée du milieu de déleucocytation permet d'obtenir des temps d'amorçage et de filtration réduits et le prémouillage des unités de filtration pendant leur stockage avant utilisation n'est plus obligatoire.

Le greffage améliore également la déleucocytation, ce qui permet une réduction du nombre de couches ainsi que de la surface du milieu de déleucocytation, donc une diminution de la perte en composant sanguin.

**Revendications**

1. Procédé de greffage d'un élément poreux pour la déleucocytation par adsorption et/ou filtration d'un fluide biologique tel que le sang ou un composant sanguin, ledit procédé comprenant les étapes prévoyant :

   - d'imprégner l'élément poreux avec une solution contenant un poly(oxyde d'éthylène) ayant des groupements terminaux fonctionnels identiques ou différents;
   - d'appliquer un traitement ionisant sur l'élément poreux imprégné de sorte à assurer un pontage covalent entre le poly(oxyde d'éthylène) et ledit élément poreux.

**2.** Procédé de greffage selon la revendication 1, **caractérisé en ce que** le poly(oxyde d'éthylène) est un macromonomère de poly(oxyde d'éthylène) dont l'un au moins des groupements terminaux est un groupement qui est polymérisable sous traitement ionisant.

**3.** Procédé de greffage selon la revendication 1 ou 2, **caractérisé en ce que** la solution d'imprégnation contient entre 0,01 et 5% en masse de poly(oxyde d'éthylène).

**4.** Procédé de greffage selon l'une quelconque des revendications 1 à 3, **caractérisé en ce que** la solution d'imprégnation comprend un solvant aqueux.

**5.** Procédé de greffage selon l'une quelconque des revendications 1 à 4, **caractérisé en ce que** le traitement ionisant est réalisé au moyen d'un plasma gazeux ou d'un faisceau d'électrons.

**6.** Procédé de greffage selon l'une quelconque des revendications 1 à 5, **caractérisé en ce que** l'imprégnation est réalisée par foulardage, notamment avec un taux d'emport compris entre 0,1% et 5% en poids de l'élément poreux.

**7.** Procédé de greffage selon l'une quelconque des revendications 2 à 6, **caractérisé en ce que** le groupement terminal polymérisable est un acrylate ou un méthacrylate.

**8.** Procédé de greffage selon la revendication 7, **caractérisé en ce que** le macromonomère est un monométhacrylate de méthoxypoly(oxyde d'éthylène).

**9.** Procédé de greffage selon l'une quelconque des revendications 1 à 8, **caractérisé en ce que** le poly(oxyde d'éthylène) présente une masse molaire supérieure ou égale à 500 g/mol.

**10.** Procédé de greffage selon la revendication 9, **caractérisé en ce que** le poly(oxyde d'éthylène) présente une masse molaire comprise entre 800 et 2000 g/mol.

**11.** Elément poreux pour la déleucocytation par adsorption et/ou filtration d'un fluide biologique tel que le sang ou un composant sanguin, ledit élément ayant été greffé avec un poly(oxyde d'éthylène) par mise en oeuvre du procédé selon l'une quelconque des revendications 1 à 10, une extrémité dudit poly(oxyde d'éthylène) étant liée de façon covalente audit élément poreux.

**12.** Elément poreux selon la revendication 11, **caractérisé en ce que** le diamètre moyen des pores est compris entre 3 et 20 $\mu$m.

**13.** Elément poreux selon la revendication 11 ou 12, **caractérisé en ce qu'**il comprend au moins une couche fibreuse.

**14.** Elément poreux selon la revendication 13, **caractérisé en ce que** la couche fibreuse est une couche de non-tissé.

**15.** Elément poreux selon la revendication 13 ou 14, **caractérisé en ce que** la couche fibreuse est réalisée en polyester, notamment en polybutylène téréphtalate.

**16.** Elément poreux selon l'une quelconque des revendications 11 à 15, **caractérisé en ce qu'**il présente une tension superficielle critique de mouillabilité comprise entre 80 et 110 dyn/cm.

**17.** Unité de filtration d'un fluide biologique tel que le sang ou un composant sanguin comprenant une enveloppe extérieure munie d'au moins un orifice d'entrée et d'au moins un orifice de sortie, l'enveloppe renfermant un milieu de déleucocytation interposé entre lesdits orifices, ledit milieu de déleucocytation comprenant au moins un élément poreux selon l'une quelconque des revendications 11 à 16.

**18.** Unité de filtration selon la revendication 17, **caractérisée en ce qu'**elle comprend en outre un pré-filtre et/ou post-filtre qui sont réalisés sous la forme d'au moins une couche de non-tissé et qui sont disposés respectivement côté amont et côté aval du milieu de déleucocytation.

**19.** Système à poches (8) pour la déleucocytation d'un fluide biologique tel que le sang ou le composant sanguin, **caractérisé en ce qu'**il comprend :

- un connecteur (9) destiné à être relié à une poche de collecte contenant le fluide biologique, ledit connecteur étant connecté, par l'intermédiaire d'une tubulure (10), à un orifice d'entrée d'une unité de filtration (11) selon la revendication 17 ou 18 ; et
- une poche de recueil du filtrat (12), ladite poche étant reliée, par l'intermédiaire d'une tubulure (13) et au niveau d'un orifice d'entrée, à un orifice de sortie de ladite unité de filtration (11).

**20.** Système à pochés pour la déleucocytation d'un fluide biologique tel que le sang ou un composant sanguin, **caractérisé en ce qu'**il comprend :

- une poche de collecte (14) du fluide biologique, ladite poche étant reliée, par l'intermédiaire d'une tubulure (10) et au niveau d'un orifice de sortie, à un orifice d'entrée d'une unité de filtration (11) selon la revendication 17 ou 18 ; et
- une poche de recueil du filtrat (12), ladite poche étant reliée, par l'intermédiaire d'une tubulure (13) et au niveau d'un orifice d'entrée, à un orifice de sortie de ladite unité de filtration (11).

## Claims

**1.** Method for grafting a porous element for removing leukocytes by means of adsorption and/or filtration from a biological fluid such as blood or a blood component, said method comprising steps for:

- impregnating the porous element with a solution containing a poly(ethylene oxide) having identical or different terminal functional groups;
- applying an ionising treatment on the impregnated porous element so as to ensure covalent coupling between the poly(ethylene oxide) and said porous element.

**2.** Grafting method according to claim 1, **characterised in that** the poly(ethylene oxide) is a macromonomer of poly (ethylene oxide) wherein at least one of the terminal groups is a group that is suitable for polymerisation under ionising treatment.

**3.** Grafting method according to claim 1 or 2, **characterised in that** the impregnation solution contains between 0.01 and 5% by mass of poly(ethylene oxide).

**4.** Grafting method according to any of claims 1 to 3, **characterised in that** the impregnation solution comprises an aqueous solvent.

**5.** Grafting method according to any of claims 1 to 4, **characterised in that** the ionising treatment is carried out by means of a gaseous plasma or an electron beam.

**6.** Grafting method according to any of claims 1 to 5, **characterised in that** the impregnation is carried out by means of padding, particularly with a payload between 0.1% and 5% by weight of the porous element.

**7.** Grafting method according to any of claims 2 to 6, **characterised in that** the terminal group suitable for polymerisation is an acrylate or a methacrylate.

**8.** Grafting method according to claim 7, **characterised in that** the macromonomer is a monomethacrylate of methoxypoly(ethylene oxide).

**9.** Grafting method according to any of claims 1 to 8, **characterised in that** the poly(ethylene oxide) has a molar mass greater than or equal to 500 g/mol.

**10.** Grafting method according to claim 9, **characterised in that** the poly(ethylene oxide) has a molar mass between 800 and 2000 g/mol.

**11.** Porous element for removing leukocytes by means of adsorption and/or filtration from a biological fluid such as blood or a blood component, said element having been grafted with a poly(ethylene oxide) using the method according to any of claims 1 to 10, one end of said poly(ethylene oxide) being covalently bonded with said porous element.

**12.** Porous element according to claim 11, **characterised in that** the mean pore diameter is between 3 and 20 μm.

**13.** Porous element according to claim 11 or 12, **characterised in that** it comprises at least one fibrous layer.

**14.** Porous element according to claim 13, **characterised in that** the fibrous layer is a layer of non-woven material.

**15.** Porous element according to claim 13 or 14, **characterised in that** the fibrous layer is made of polyester, particularly polybutylene terephthalate.

**16.** Porous element according to any of claims 11 to 15, **characterised in that** it has a critical surface tension of wettability between 80 and 110 dyn/cm.

**17.** Unit for filtering a biological fluid such as blood or a blood component comprising an outer casing provided with at least one inlet orifice and at least one outlet orifice, the casing containing a leukocyte removal medium inserted between said orifices, said leukocyte removal medium comprising at least one porous element according to any of claims 11 to 16.

**18.** Filtration unit according to claim 17, **characterised in that** it further comprises a pre-filter and/or post-filter embodied in the form of at least one layer of non-woven material and arranged at the upstream end and downstream end of the leukocyte removal medium, respectively.

**19.** Bag system (8) for removing leukocytes from a biological fluid such as blood or a blood component, **characterised in that** it comprises:

    - a connector (9) to be connected to a collection bag containing the biological fluid, said connector being connected, via a tube (10), to an inlet orifice of a filtration unit (11) according to claim 17 or 18; and
    - a bag for collecting the filtrate (12), said bag being connected, via a tube (13) and at an inlet orifice, to an outlet orifice of said filtration unit (11).

**20.** Bag system for removing leukocytes from a biological fluid such as blood or a blood component, **characterised in that** it comprises:

    - a bag (14) for collecting the biological fluid, said bag being connected, via a tube (10), to an inlet orifice of a filtration unit (11) according to claim 17 or 18; and
    - a bag for collecting the filtrate (12), said bag being connected, via a tube (13) and at an inlet orifice, to an outlet orifice of said filtration unit (11).

**Patentansprüche**

**1.** Verfahren zum Okulieren eines porösen Elements für die Entfernung der Leukozyten durch Adsorption und/oder Filtrierung eines biologischen Fluids wie z.B. Blut oder einer Blutkomponente, wobei das Verfahren die Schritte umfasst, die Folgendes vorsehen:

    - Imprägnieren des porösen Elements mit einer Lösung, die ein Polyethylenoxid mit identischen oder verschiedenen funktionellen Endgruppen aufweist;
    - Anwenden einer ionisierenden Behandlung auf das imprägnierte poröse Element, um eine kovalente Brückenbildung zwischen dem Polyethylenoxid und dem porösen Element sicherzustellen.

**2.** Verfahren zum Okulieren nach Anspruch 1, **dadurch gekennzeichnet, dass** das Polyethylenoxid ein Makromonomer aus Polyethylenoxid ist, von dem mindestens die eine der Endgruppen eine Gruppe darstellt, die bei ionisierender Behandlung polymerisierbar ist.

**3.** Verfahren zum Okulieren nach Anspruch 1 oder 2, **dadurch gekennzeichnet, dass** die Imprägnierlösung zwischen 0,01 und 5% Massegehalt an Polyethylenoxid enthält.

**4.** Verfahren zum Okulieren nach einem der Ansprüche 1 bis 3, **dadurch gekennzeichnet, dass** die Imprägnierlösung ein wässriges Lösemittel umfasst.

5. Verfahren zum Okulieren nach einem der Ansprüche 1 bis 4, **dadurch gekennzeichnet, dass** die ionisierende Behandlung mit Hilfe eines Gasplasmas oder eines Elektronenstrahls durchgeführt wird.

6. Verfahren zum Okulieren nach einem der Ansprüche 1 bis 5, **dadurch gekennzeichnet, dass** die Imprägnierung durch Klotzen durchgeführt wird, insbesondere mit einer Mitführrate, die zwischen 0,1 Gew% und 5 Gew% des porösen Elements liegt.

7. Verfahren zum Okulieren nach einem der Ansprüche 2 bis 6, **dadurch gekennzeichnet, dass** die polymerisierbare Endgruppe ein Acrylat oder ein Methacrylat ist.

8. Verfahren zum Okulieren nach Anspruch 7, **dadurch gekennzeichnet, dass** das Makromonomer ein Methoxypolyethylenoxid-Monomethacrylat ist.

9. Verfahren zum Okulieren nach einem der Ansprüche 1 bis 8, **dadurch gekennzeichnet, dass** das Polyethylenoxid eine Molekularmasse aufweist, die größer oder gleich 500 g/mol ist.

10. Verfahren zum Okulieren nach Anspruch 9, **dadurch gekennzeichnet, dass** das Polyethylenoxid eine Molekularmasse zwischen 800 und 2000 g/mol aufweist.

11. Poröses Element für die Entfernung der Leukozyten durch Adsorption und/oder Filtrierung eines biologischen Fluids wie z.B. Blut oder einer Blutkomponente, wobei das Element mit einem Polyethylenoxid durch die Durchführung des Verfahrens nach einem der Ansprüche 1 bis 10 okuliert wurde, wobei ein Ende des Polyethylenoxids auf kovalente Weise an das poröse Element gebunden ist.

12. Poröses Element nach Anspruch 11, **dadurch gekennzeichnet, dass** der mittlere Durchmesser der Poren zwischen 3 und 20 $\mu$m liegt.

13. Poröses Element nach Anspruch 11 oder 12, **dadurch gekennzeichnet, dass** es mindestens eine faserige Schicht umfasst.

14. Poröses Element nach Anspruch 13, **dadurch gekennzeichnet, dass** die faserige Schicht eine Vliesstoffschicht ist.

15. Poröses Element nach Anspruch 13 oder 14, **dadurch gekennzeichnet, dass** die faserige Schicht aus Polyester hergestellt ist, insbesondere aus Polyethylenterephtalat.

16. Poröses Element nach einem der Ansprüche 11 bis 15, **dadurch gekennzeichnet, dass** es eine kritische Oberflächenspannung der Benetzbarkeit zwischen 80 und 110 dyn/cm aufweist.

17. Einheit zur Filtration eines biologischen Fluids wie z.B. Blut oder einer Blutkomponente, umfassend eine äußere Hülle, ausgestattet mit mindestens einer Eingangsöffnung und einer Ausgangsöffnung, wobei die Hülle ein Milieu zur Entfernung der Leukozyten einschließt, das sich zwischen den Öffnungen befindet, wobei das Milieu zur Entfernung der Leukozyten mindestens ein poröses Element nach einem der Ansprüche 11 bis 16 umfasst.

18. Einheit zur Filtrierung nach Anspruch 17, **dadurch gekennzeichnet, dass** sie außerdem eine Vorfilter und/oder einen Nachfilter umfasst, die in der Form mindestens einer Schicht Vliesstoff durchgeführt sind und die jeweils an der dem Milieu zur Entfernung der Leukozyten vorgelagerten und nachgelagerten Seite angeordnet sind.

19. System mit Beuteln (8) zur Entfernung der Leukozyten eines biologischen Fluids wie z.B. Blut oder einer Blutkomponente, **dadurch gekennzeichnet, dass** es Folgendes umfasst:

   - ein Verbindungselement (9), das dazu vorgesehen ist, mit einem Sammelbeutel verbunden zu werden, die das biologische Fluid enthält, wobei das Verbindungselement mit Hilfe eines Rohransatzes (10) mit einer Eingangsöffnung einer Einheit zur Filtrierung (11) nach Anspruch 17 oder 18 verbunden ist; und
   - einen Beutel zur Aufnahme des Filtrats (12), wobei der Beutel mit Hilfe eines Rohransatzes (13) und auf dem Niveau einer Eingangsöffnung mit einer Ausgangsöffnung der Einheit zur Filtrierung (11) verbunden ist.

20. System mit Beuteln zur Entfernung der Leukozyten eines biologischen Fluids wie z.B. Blut oder einer Blutkomponente, **dadurch gekennzeichnet, dass** es Folgendes umfasst:

- einen Beutel zur Aufnahme (14) des biologischen Fluids, wobei der Beutel mit Hilfe eines Rohransatzes (10) und auf dem Niveau einer Ausgangsöffnung mit einer Eingangsöffnung einer Einheit zur Filtrierung (11) nach Anspruch 17 oder 18 verbunden ist.
- einen Beutel zur Aufnahme des Filtrats (12), wobei der Beutel mit Hilfe eines Rohransatzes (13) und auf der Höhe einer Eingangsöffnung mit einer Ausgangsöffnung der Einheit zur Filtrierung (11) verbunden ist.

Fig. 1

Fig. 2

Fig. 3

**Fig. 4**

**Fig. 5**

**Fig. 6**

Fig. 7

## RÉFÉRENCES CITÉES DANS LA DESCRIPTION

**Documents brevets cités dans la description**

- EP 0606646 A1
- US 20060207937 A
- WO 2007054638 A
- EP 811412 A
- WO 8903717 A

**Littérature non-brevet citée dans la description**

- *Biomacromolecules,* 2004, vol. 5, 1280-1287